# EUROPEAN PATENT APPLICATION

(11) **EP 4 506 461 A1**
(43) Date of publication of application: **12.02.2025**
(21) Application number: 23784180.4
(22) Date of filing: 24.03.2023
(51) Int. Cl.: C12N 15/90, C12N 9/22, C12N 5/10

(54) **METHOD FOR REPAIRING HBA2 GENE MUTATIONS BY SINGLE BASE EDITING AND USE THEREOF**

(30) Priority: 06.04.2022 CN 202210355452
(71) Applicant: Guangzhou Reforgene Medicine Co., Ltd., Guangzhou, Guangdong 510535 (CN)
(72) Inventor: LIANG, Junbin, Guangzhou, Guangdong 510535 (CN); XU, Hui, Guangzhou, Guangdong 510535 (CN); YE, Lu, Guangzhou, Guangdong 510535 (CN); LIN, Simiao, Guangzhou, Guangdong 510535 (CN)
(74) Representative: Studio Torta S.p.A.
(86) International application number: PCT/CN2023/083728
(87) International publication number: WO 2023/193616

(57) **Abstract**

A method for repairing HBA2 gene mutations by single base editing and use thereof, belonging to the technical field of gene editing. The method comprises the following steps: contacting a single base editor and gRNA with an HBA gene sequence to be edited and repaired, deaminating the codon target cytosine base at CD142 in the HBA gene sequence, and converting the cytosine into thymine. According to the method, pathogenic mutation sites can be accurately edited without generating double-strand breaks and affecting chromatin conformation and genome stability, without generating random insertions of large-fragment genes into genomes, which have a low influence on cancer gene activation or tumor-inhibiting gene inactivation, and without generating random insertions/deletions at other sites of a genome. The expression of the repaired target gene is regulated by all regulatory elements of natural HBA2; therefore, the method is very safe and can also better balance the expression of α/β globin.

## Description

### TECHNICAL FIELD

The present disclosure relates to the technical field of gene editing, and more particularly, to a method for repairing an HBA2 gene mutation by single base editing and a use thereof.

### BACKGROUND

Thalassemia is a hereditary hemolytic anemia that is widely distributed in southern China, with the Guangxi region being the most severely affected. Clinically, based on genotyping, there are two main types: α-thalassemia and β-thalassemia. The mutations that cause α-thalassemia are classified into deletional and non-deletional mutations. Non-deletional α-thalassemia is caused by a "point mutation" in the α-gene nucleotides. In regions such as Guangdong, Guangxi, and Sichuan, non-deletional α-thalassemia accounts for 35% to 60% of α-thalassemia cases. The three most common types of non-deletional α-thalassemia in China are Hb-CS (CD142), Hb-QS (CD125), and Hb-WS (CD122). Patients with Hb-CS tend to have the most severe symptoms, with low hemoglobin levels and a poor survival rate.

Haemoglobin-Constant Spring (Hb-CS, α142, Term→Gln, TAA>CAA (α2), α^{CS}α/) is a non-deletional form of α-thalassemia (α-Thal) with a nucleotide substitution at the stop codon CD142 (UAA>CAA) of the α2-globin gene (HBA2). When Hb-CS is complex with α2 and α1 double-deletion thalassemia mutations (e.g., α^{SEA} α^{MED}, α^{THAI}), leaving only one normal α1-globin gene (HBA1), the genotype is α^{CS}α/--, whose clinical manifestation and hemogram are similar to those of hemoglobin H disease (HbH), referred to as CS-type HbH disease (HbH-CS). Furthermore, individuals affected by HbH-CS disease typically exhibit more severe anemia and are prone to significant hepatosplenomegaly, especially compared to those with a three-gene deletion of the α-globin gene (--/-α), and patients with HbH-CS have a greater need for blood transfusion.

Hematopoietic stem cell (HSC) transplantation is an efficient means and the only way to cure severe thalassemia, while the lack of HLA-matched healthy donors, immune complications, and safety concerns with viral vectors has limited its use.

In conventional techniques, there are some gene therapy methods for α-thalassemia, such as gene overexpression of α-globin, ζ-globin, *etc.* However, these methods present significant safety concerns, such as random integration of overexpressed globin gene copies into thousands of different sites in the genome, leading to insertional mutagenesis and the potential for malignancies. Moreover, these techniques have not yet been proven to be effective or safe in patient cells, animal experiments, or clinical trials.

The Hb-CS site can also be repaired by nuclease cleavage of the target site as well as by homology-directed gene repair. However, the method currently has low efficiency, which is insufficient to achieve therapeutic levels. Meanwhile, the cleavage approach can introduce indels, resulting in unnatural α-globin mutations, which also pose safety risks. Moreover, the cleavage approach may have certain off-target risks, such as random insertions/deletions at other loci, or genomic double-strand breaks that may lead to changes in chromatin conformation, thereby affecting genome stability.

### SUMMARY

Based on this, it is necessary to address the above issues by providing a method for repairing an HBA2 gene mutation by single base editing. By using the method, pathogenic mutation sites can be precisely edited without generating double-strand breaks or affecting chromatin conformation and genome stability, without generating random insertions of large-fragment genes into genomes, which have a low impact on oncogene activation or tumor suppressor gene inactivation, and without generating random insertions/deletions at other sites in the genome. Meanwhile, the expression of a repaired target gene is regulated by all regulatory elements of natural HBA2, which is high in safety and is more capable of balancing the expression of α/β globin.

The present disclosure discloses a method for repairing an HBA2 gene mutation by single base editing, comprising the following steps: contacting a single base editor and a gRNA with an HBA2 gene sequence to be edited and repaired, deaminating the target cytosine base of mutated codon (CAA) at CD142 of HBA2 gene, to convert cytosine to thymine (TAA).

The method for repairing an HBA2 gene mutation by single base editing takes advantage of the feature that the single base editor can precisely modify a single base in the genome without causing DNA double-strand breaks by contacting the single base editor and gRNA with the HBA2 gene that has undergone a pathogenic mutation and deaminating the target cytosine (C) base of the HBA2 gene, which results in the conversion of cytosine (C) to thymine (T) at the target site of the polynucleotide encoding α-globin, and provides a more efficient and safer method for repairing the Hb-CS mutation.

It can be understood that the method for repairing an HBA2 gene mutation by single base editing *i.e.,* a method for editing the polynucleotide encoding α-globin, comprises contacting an HBA2 polynucleotide sequence encoding α-globin with a fusion protein and a guide nucleotide sequence (e.g., gRNA), wherein the fusion protein comprises a programmable DNA-binding protein domain that binds to the guide nucleotide sequence, and a cytosine deaminase domain. The guide nucleotide sequence targets the fusion protein to the target cytosine (C) base in the polynucleotide encoding HBA2. The subsequent contacting leads to deamination of the target C base by the fusion protein, which results in the conversion of cytosine (C) to thymine (T) in the polynucleotide encoding HBA.

In some embodiments, the method for repairing an HBA2 gene mutation by single base editing is used for non-therapeutic purposes, such as scientific research.

The present disclosure also discloses a use of the method for repairing an HBA2 gene mutation by single base editing as described above in the preparation of a medicament for the treatment of α-thalassemia.

The present disclosure also discloses a composition for repairing an HBA2 gene mutation, comprising a single base editor and a gRNA, wherein
the single base editor comprises a target nucleic acid-binding structural unit and a deaminase structural unit, wherein the deaminase structural unit comprises a cytosine deaminase active domain;
the gRNA comprises a guide sequence and a scaffold sequence that binds to the single base editor, wherein the guide sequence targets the mutated cytosine base at CD142 codon (CAA) of the HBA2 gene.

It can be understood that the "target" as described above means that the gRNA guides the single base editor to the vicinity of the mutated cytosine at CD142 codon of the HBA2 gene to edit the cytosine. Typically, the guide sequence binds to the antisense strand of the mutated cytosine base at CD142 codon of the HBA2 gene.

The target nucleic acid-binding structural unit in the single base editor is a structure that can accurately localize and bind to a target nucleic acid editing domain, which may be a nucleotide or a protein polypeptide. It can be understood that in some embodiments, organic linkage means that the target nucleic acid-binding structural unit is connected to the deaminase structural unit or a partially active domain thereof by a peptide bond or linker peptide, thereby forming a fusion protein. Moreover, the fusion protein may comprise one or more uracil-DNA glycosylase inhibitor (UGI) domains, one or more nuclear localization signals (NLS), one or more Gam proteins, *etc.,* to improve the base repair efficiency, provided only that it has the function of converting the cytosine (C) base to thymine (T) through deamination.

In some embodiments, the deaminase structural unit comprised in the single base editor may be a deaminase protein or an active domain thereof, a polypeptide that can recruit the deaminase protein, or a nucleotide-binding motif that can recruit the deaminase protein, wherein the nucleotide-binding motif may be linked to a gRNA.

The following are examples of a cytosine deaminase that can achieve the deamination function as described above, including, but not limited to, APOBEC1 deaminase, APOBEC2 deaminase, APOBEC3A deaminase, APOBEC3B deaminase, APOBEC3C deaminase, APOBEC3D deaminase, APOBEC3F deaminase, APOBEC3G deaminase, APOBEC3H deaminase, APOBEC4 deaminase, activation-induced deaminase (AID), pmCDA1, and a variant or combination thereof. Also included is a cytosine deaminase variant, which may be, for example, Anc689 APOBEC, YE1 APOBEC, *etc.*

The gRNA in the composition may be a single-molecule gRNA, a dual-molecule gRNA, or a multi-molecule gRNA. The gRNA can be replaced with other RNA molecules comprising a guide sequence that can bind to the target site by complementary pairing and a scaffold sequence that is connected to or binds to the single base editor.

In some embodiments, the target nucleic acid-binding structural unit comprises a programmable nucleic acid-binding protein, which refers to a nucleic acid-binding protein that can be programmed to target any desired nucleotide sequence within the genome. In order to program the nucleic acid-binding protein to bind to the desired nucleotide sequence, the nucleic acid-binding protein can be modified to alter its binding specificity, such as zinc finger DNA-binding domain, zinc finger nuclease (ZFN), or transcription activator-like effector protein (TALE).

In some embodiments, the target nucleic acid-binding structural unit is selected from: CRISPR/Cas nuclease, ZFN, TALEN, and Argonaute (AGO protein).

The Argonaute refers to AGO proteins, which can bind to siRNA and cleave the target nucleotide through the binding of siRNA to mRNA, exhibiting the characteristics of "programmable nucleic acid-binding protein" of the present disclosure.

It can be understood that the CRISPR/Cas nuclease in the target nucleic acid-binding structural unit is preferably a protein whose catalytic activity is partially or completely inactivated and whose localization function alone is utilized.

In some embodiments, the guide nucleotide sequence-programmable nucleic acid-binding protein may be a DNA-binding protein or an RNA-binding protein.

In some embodiments, the CRISPR/Cas nuclease is at least one selected from: Cas9, CasX, CasY, Cpfl, C2c1, cas12b2, cas12h, cas12i, cas12g, C2c2, C2c3, C2c4, C2c5, C2c6, C2c7, c2c8, c2c9, c2c10, cas14a, cas14b, cas14c, Cas13d, and a variant thereof.

In some embodiments, the CRISPR/Cas nuclease is at least one selected from: Cas9, CasX, CasY, Cpfl, C2c1, cas12b2, cas12h, cas12i, cas12g, C2c3, C2c4, C2c5, c2c8, c2c9, c2c10, cas14a, cas14b, cas14c, and a variant thereof.

In some embodiments, the CRISPR/Cas nuclease has nickase activity.

In some embodiments, the CRISPR/Cas nuclease is a Cas9 variant with nickase activity.

In some embodiments, the target nucleic acid-binding structural unit is selected from: a nuclease-inactive Cas9 (dCas9) domain, a nuclease-inactive Cpf1 domain, a nuclease-inactive Argonaute domain, a nuclease-partially inactive Cas9 (nCas9) domain, or a variant or combination thereof.

In some embodiments, the CRISPR/Cas nuclease is selected from a Cas9 nickase variant (nCas9).

It can be understood that the programmable nucleic acid-binding protein may have an optimal PAM (protospacer adjacent motif) site, or may have no optimal PAM site or no PAM site restriction.

In some embodiments, the target nucleic acid-binding structural unit has no PAM site restriction.

In some embodiments, the target nucleic acid-binding structural unit has an optimal PAM site. For example, the optimal PAM site is NGG, NG, NNNNCC, NAA, NAAN, NGGNG, NNNNGATT, NNNNCNDD, NNNNCAAA, NNNNCRAA, NNAGAAW, NNGRRN, TTN, TTTV/TTTN, ATTN, or TTCN (N represents A/T/C/G, R represents A/G, W represents A/T, and V represents A/C/G).

In some embodiments, the CRISPR/Cas nuclease is at least one selected from: a SpRY variant, a NG-nCas9 variant, and a NGG-nCas9 variant. The SpRY variant is a SpRY nuclease variant without PAM site restriction, the NG-nCas9 variant is nCas9 with an optimal PAM of NG, and the NGG-nCas9 variant is nCas9 with an optimal PAM of NGG.

In some embodiments, the cytosine deaminase domain comprises an apolipoprotein B mRNA-editing complex (APOBEC) family deaminase.

In some embodiments, the deaminase structural unit is selected from: APOBEC1 deaminase, APOBEC2 deaminase, APOBEC3A deaminase, APOBEC3B deaminase, APOBEC3C deaminase, APOBEC3D deaminase, APOBEC3F deaminase, APOBEC3G deaminase, APOBEC3H deaminase, APOBEC4 deaminase, activation-induced deaminase (AID), pmCDA1, and a variant or combination thereof.

In some embodiments, the target nucleic acid-binding structural unit is organically connected to the deaminase structural unit. The target nucleic acid-binding structural unit in the single base editor is a structure that can accurately localize and bind to a target nucleic acid editing domain, which may be a nucleotide or a protein polypeptide.

It can be understood that in some embodiments, the target nucleic acid-binding structural unit is organically connected to the deaminase structural unit or a partially active domain thereof by a peptide bond or linker peptide, thereby forming a fusion protein. Moreover, the fusion protein may comprise one or more uracil-DNA glycosylase inhibitor (UGI) domains, one or more nuclear localization signals (NLS), one or more Gam proteins, *etc.,* to improve the base repair efficiency, provided only that it has the function of converting the cytosine (C) base to thymine (T) through deamination.

In some embodiments, the cytosine deaminase variant is an Anc689 APOBEC variant.

In some embodiments, the cytosine deaminase variant is a W90Y/R126E APOBEC variant.

In some embodiments, the single base editor is at least one selected from: SpRY-CBE, AncBE, and YE1-CBE.

The single base editor is a fusion protein formed by organic linkage of the target nucleic acid-binding structural unit and the partially active domain of the deaminase structural unit by a peptide bond or linker peptide.

The SpRY-CBE is a SpRY nuclease without optimal PAM site restriction connected to a cytidine deaminase (SpRY-CBE), preferably a SpRY nuclease-cytidine deaminase connected to two UGIs, preferably a SpRY nuclease-cytidine deaminase-2×UGI connected to two or more NLSs, preferably a CBE4max-SpRY base editor.

The cytidine deaminase of AncBE is an Anc689 APOBEC variant, preferably a nCas9 nuclease with impaired protein activity in the target nucleic acid-binding structural unit, preferably nCas9-Anc689 APOBEC connected to two UGIs, or nCas9-Anc689 APOBEC-2×UGI connected to two or more NLSs, more preferably an AncBE4max base editor.

The YE1-CBE is YE1 APOBEC in which APOBEC comprises W90Y + R126E mutations, preferably a programmable nucleic acid-binding protein connected to YE1 APOBEC, preferably a programmable nucleic acid-binding protein-YE1 APOBEC-2×UGI connected to two or more NLSs, preferably a YE1-BE4max base editor.

In some embodiments, the guide sequence of the gRNA binds to or is reverse complementary to a target region of Chr16: 173548-173648. It can be understood that the region refers to the region where the gRNA is intended to guide the single base editor to the mutated cytosine at CD142 codon of the HBA2 gene. Due to single nucleotide polymorphism (SNP) in organisms, there may be some natural mutations in the region. The guide sequence of the gRNA can be adjusted or designed based on these natural mutations, or allow for 1 to 5 base mismatches while still binding to the region, thereby achieving the purpose of the present disclosure.

In some embodiments, the guide sequence of the gRNA binds to or is reverse complementary to a target region of Chr16: 173589-173613, Chr16: 173588-173612, Chr16: 173586-173610, Chr16: 173590-173614, Chr16: 173587-173611, Chr16: 173590-173609, or Chr16: 173596-173615.

In some embodiments, the base at the 3' end of the guide sequence of the gRNA binds to the base at the 5' end of the antisense strand of the target region. The binding of the base at the 3' end of the guide sequence of the gRNA to the base at the 5' end of the antisense strand of the genomic region defines the Hb-CS mutation localization, *i.e.,* limits the distance between the Hb-CS mutation base pair and the 3' end of the gRNA or the PAM site.

In some embodiments, the guide sequence of the gRNA is reverse complementary to or contains 1 to 5 mismatches with Chr16: 173594-173613, Chr16: 173599-173613, Chr16: 173598-173613, Chr16: 173597-173613, Chr16: 173596-173613, Chr16: 173595-173613, Chr16: 173593-173613, Chr16: 173592-173613, Chr16: 173591-173613, Chr16: 173590-173613, Chr16: 173589-173613, Chr16: 173593-173612, Chr16: 173598-173612, Chr16: 173597-173612, Chr16: 173596-173612, Chr16: 173595-173612, Chr16: 173594-173612, Chr16: 173592-173612, Chr16: 173591-173612, Chr16: 173590-173612, Chr16: 173589-173612, Chr16: 173588-173612, Chr16: 173591-173610, Chr16: 173596-173610, Chr16: 173595-173610, Chr16: 173594-173610, Chr16: 173593-173610, Chr16: 173592-173610, Chr16: 173590-173610, Chr16: 173589-173610, Chr16: 173588-173610, Chr16: 173587-173610, Chr16: 173586-173610, Chr16: 173595-173614, Chr16: 173600-173614, Chr16: 173599-173614, Chr16: 173598-173614, Chr16: 173597-173614, Chr16: 173596-173614, Chr16: 173594-173614, Chr16: 173593-173614, Chr16: 173592-173614, Chr16: 173591-173614, Chr16: 173590-173614, Chr16: 173592-173611, Chr16: 173596-173611, Chr16: 173596-173611, Chr16: 173595-173611, Chr16: 173594-173611, Chr16: 173593-173611, Chr16: 173591-173611, Chr16: 173590-173611, Chr16: 173589-173611, Chr16: 173588-173611, Chr16: 173587-173611, Chr16: 173590-173609, Chr16: 173594-173609, Chr16: 173596-173615, or Chr16: 173600-173615.

In some embodiments, the guide sequence of the gRNA is reverse complementary to or contains 1 to 5 mismatches with Chr16: 173594-173613, Chr16: 173598-173613, Chr16: 173597-173613, Chr16: 173596-173613, Chr16: 173595-173613, Chr16: 173593-173613, Chr16: 173592-173613, Chr16: 173591-173613, Chr16: 173590-173613, Chr16: 173589-173613, Chr16: 173593-173612, Chr16: 173597-173612, Chr16: 173596-173612, Chr16: 173595-173612, Chr16: 173594-173612, Chr16: 173592-173612, Chr16: 173591-173612, Chr16: 173590-173612, Chr16: 173589-173612, Chr16: 173588-173612, Chr16: 173591-173610, Chr16: 173595-173610, Chr16: 173594-173610, Chr16: 173593-173610, Chr16: 173592-173610, Chr16: 173590-173610, Chr16: 173589-173610, Chr16: 173588-173610, Chr16: 173587-173610, Chr16: 173586-173610, Chr16: 173595-173614, Chr16: 173599-173614, Chr16: 173598-173614, Chr16: 173597-173614, Chr16: 173596-173614, Chr16: 173594-173614, Chr16: 173593-173614, Chr16: 173592-173614, Chr16: 173591-173614, Chr16: 173590-173614, Chr16: 173592-173611, Chr16: 173596-173611, Chr16: 173595-173611, Chr16: 173594-173611, Chr16: 173593-173611, Chr16: 173591-173611, Chr16: 173590-173611, Chr16: 173589-173611, Chr16: 173588-173611, or Chr16: 173587-173611.

In some embodiments, the guide sequence of the gRNA is reverse complementary to or contains 1 to 5 mismatches with Chr16: 173594-173613, Chr16: 173598-173613, Chr16: 173597-173613, Chr16: 173596-173613, Chr16: 173595-173613, Chr16: 173593-173613, Chr16: 173592-173613, Chr16: 173591-173613, Chr16: 173590-173613, or Chr16: 173589-173613.

In some embodiments, the guide sequence of the gRNA is reverse complementary to or contains 1 to 5 mismatches with Chr16: 173593-173612, Chr16: 173597-173612, Chr16: 173596-173612, Chr16: 173595-173612, Chr16: 173594-173612, Chr16: 173592-173612, Chr16: 173591-173612, Chr16: 173590-173612, Chr16: 173589-173612, or Chr16: 173588-173612.

In some embodiments, the guide sequence of the gRNA is reverse complementary to or contains 1 to 5 mismatches with Chr16: 173591-173610, Chr16: 173595-173610, Chr16: 173594-173610, Chr16: 173593-173610, Chr16: 173592-173610, Chr16: 173590-173610, Chr16: 173589-173610, Chr16: 173588-173610, Chr16: 173587-173610, or Chr16: 173586-173610.

In some embodiments, the guide sequence of the gRNA is reverse complementary to or contains 1 to 5 mismatches with Chr16: 173595-173614, Chr16: 173599-173614, Chr16: 173598-173614, Chr16: 173597-173614, Chr16: 173596-173614, Chr16: 173594-173614, Chr16: 173593-173614, Chr16: 173592-173614, Chr16: 173591-173614, or Chr16: 173590-173614.

In some embodiments, the guide sequence of the gRNA is reverse complementary to or contains 1 to 5 mismatches with Chr16: 173592-173611, Chr16: 173596-173611, Chr16: 173595-173611, Chr16: 173594-173611, Chr16: 173593-173611, Chr16: 173591-173611, Chr16: 173590-173611, Chr16: 173589-173611, Chr16: 173588-173611, or Chr16: 173587-173611.

In some embodiments, the 3' end of the guide sequence of the gRNA is at a distance of C12 to C18 from the codon cytosine at CD142 of the HBA2 gene sequence.

The distance between the 3' end of the guide sequence and the cytosine is also the distance between the Hb-CS mutation site and the PAM. The present disclosure has discovered through research that gRNAs with a distance of C12 to C18 can repair the Hb-CS mutation.

"Hb-CS mutation localization" refers to the localization of the Hb-CS mutation site in the single base editor-gRNA, *i.e.,* the distance between the 3' end of the guide sequence of the gRNA and the codon cytosine at CD142 of the HBA2 gene sequence, and specifically, in some embodiments, is represented by the position of the Hb-CS mutation base in the guide sequence of the gRNA, *i.e.,* the x-th nucleotide from the 3' end of the guide sequence, or the x-th nucleotide from the PAM site (in cases where a PAM site is present), abbreviated as Cx.

In some embodiments, the 3' end of the guide sequence of the gRNA is at a distance of C13 to C18 from the codon cytosine at CD142 of the HBA2 gene sequence.

In some embodiments, the 3' end of the guide sequence of the gRNA is at a distance of C13 to C17 from the codon cytosine at CD142 of the HBA2 gene sequence. The present disclosure has demonstrated through research that, although gRNAs with a distance of C12 to C18 can repair the Hb-CS mutation, gRNAs with a distance of C13 to C17 have a better effect compared to those with a distance of C12 or C18.

In some embodiments, the single base editor is SpRY-CBE, and the 3' end of the guide sequence of the gRNA is at a distance of C13 to C17 from the codon cytosine at CD142 of the HBA2 gene sequence.

Using the single base editor SpRY-CBE in combination with the gRNA results in higher editing efficiency.

In some embodiments, the single base editor is SpRY-CBE, and the 3' end of the guide sequence of the gRNA is at a distance of C14 to C17 from the codon cytosine at CD142 of the HBA2 gene sequence.

Using the single base editor SpRY-CBE in combination with the gRNA results in higher editing efficiency.

In some embodiments, the single base editor is SpRY-CBE, and the 3' end of the guide sequence of the gRNA is at a distance of C15 to C17 from the codon cytosine at CD142 of the HBA2 gene sequence.

Using the single base editor SpRY-CBE in combination with the gRNA results in lower off-target editing probability.

In some embodiments, the single base editor is AncBE, and the 3' end of the guide sequence of the gRNA is at a distance of C13 to C17 from the codon cytosine at CD142 of the HBA2 gene sequence.

In some embodiments, the single base editor is AncBE, and the 3' end of the guide sequence of the gRNA is at a distance of C13 to C16 from the codon cytosine at CD142 of the HBA2 gene sequence.

In some embodiments, the single base editor is AncBE, and the 3' end of the guide sequence of the gRNA is at a distance of C13 or C16 from the codon cytosine at CD142 of the HBA2 gene sequence.

Using the single base editor AncBE in combination with the gRNA results in higher editing efficiency.

In some embodiments, the single base editor is AncBE, and the 3' end of the guide sequence of the gRNA is at a distance of C16 from the codon cytosine at CD142 of the HBA2 gene sequence.

Using the single base editor AncBE in combination with the gRNA results in lower off-target editing probability.

In some embodiments, the single base editor is YE1-CBE, and the 3' end of the guide sequence of the gRNA is at a distance of C13 to C17 from the codon cytosine at CD142 of the HBA2 gene sequence.

In some embodiments, the single base editor is YE1-CBE, and the 3' end of the guide sequence of the gRNA is at a distance of C14 to C17 from the codon cytosine at CD142 of the HBA2 gene sequence.

In some embodiments, the single base editor is YE1-CBE, and the 3' end of the guide sequence of the gRNA is at a distance of C14 to C16 from the codon cytosine at CD142 of the HBA2 gene sequence.

Using the single base editor YE1-CBE in combination with the gRNA results in higher editing efficiency.

In some embodiments, the single base editor is YE1-CBE, and the 3' end of the guide sequence of the gRNA is at a distance of C15 to C16 from the codon cytosine at CD142 of the HBA2 gene sequence. Using the single base editor YE1-CBE in combination with the gRNA results in lower off-target editing probability.

In some embodiments, the length of the guide sequence of the gRNA is 16 bp or more.

Considering the gRNA structure and the design of the guide sequence to match the target nucleic acid region, the length of the guide sequence of the gRNA should be at least 16 bp to ensure precise localization for single base repair.

In some embodiments, the length of the guide sequence of the gRNA is 16 to 25 bp.

In some embodiments, the length of the guide sequence of the gRNA is 17 to 25 bp. Setting the length of the guide sequence of the gRNA within the above range results in good editing and repair efficiency.

In some embodiments, the target DNA nucleotide sequence encoding the guide sequence of the gRNA is selected from a basic sequence shown in SEQ ID NO: 1 to SEQ ID NO: 62 and a mismatched sequence comprising 1 to 5 base mismatches with the basic sequence.

In some embodiments, the number of mismatched sites of the mismatched sequence is less than 5 nucleotides. For example, the guide sequence of the gRNA may comprise a mismatch of 1, 2, 3, 4, or 5 nucleotides.

In some embodiments, the number of mismatched sites of the mismatched sequence is less than 3 nucleotides, allowing for 1 to 3 base mismatches. The present disclosure has demonstrated through experiments that the gRNA with a mismatch of 1 to 3 nucleotides still has Hb-CS mutation repair efficiency.

The mismatched site is preferably not located at the Hb-CS site and is preferably located at the front section (near the 5' end) or middle region of the guide sequence of the gRNA. Further preferably, the mismatch is at least 8 nucleotides, more preferably at least 10 nucleotides away from the 3' end of the guide sequence or the PAM site.

In some embodiments, the basic sequence is selected from a sequence shown in SEQ ID NO: 1 to SEQ ID NO: 58. Using the gRNA results in higher editing and repair efficiency.

In some embodiments, the single base editor is SpRY-CBE, and the basic sequence is selected from a sequence shown in SEQ ID NO: 1, SEQ ID NO: 3 to SEQ ID NO: 15, SEQ ID NO: 17 to SEQ ID NO: 26, SEQ ID NO: 28 to SEQ ID NO: 37, SEQ ID NO: 39 to SEQ ID NO: 48, and SEQ ID NO: 50 to SEQ ID NO: 58. Using the gRNA in combination with the single base editor results in lower off-target editing probability.

In some embodiments, the single base editor is AncBE, and the basic sequence is selected from a sequence shown in SEQ ID NO: 1 to SEQ ID NO: 14 and SEQ ID NO: 26 to SEQ ID NO: 36.

In some embodiments, the basic sequence is selected from a sequence shown in SEQ ID NO: 1, SEQ ID NO: 3 to SEQ ID NO: 14, SEQ ID NO: 26, and SEQ ID NO: 28 to SEQ ID NO: 36.

In some embodiments, the single base editor is YE1-CBE, and the basic sequence is selected from a sequence shown in SEQ ID NO: 1 to SEQ ID NO: 25 and SEQ ID NO: 48 to SEQ ID NO: 58. Using the gRNA in combination with the single base editor results in lower non-target editing probability.

In some embodiments, the basic sequence is selected from a sequence shown in SEQ ID NO: 1, SEQ ID NO: 3 to SEQ ID NO: 15, SEQ ID NO: 16 to SEQ ID NO: 25, SEQ ID NO: 48, and SEQ ID NO: 50 to SEQ ID NO: 58.

The present disclosure also discloses a gRNA for repairing an HBA2 gene mutation by single base editing, comprising the gRNA as described above.

The present disclosure also discloses a cell comprising the composition for repairing an HBA2 gene mutation.

In some embodiments, the cell is a cell line and/or a primary cell.

In some embodiments, the cell is a hematopoietic stem/progenitor cell, an induced pluripotent stem cell, or an erythroid progenitor cell with the potential to induce differentiation, transdifferentiation, or reprogramming into a mature red blood cell.

In some embodiments, somatic cells containing the Hb-CS mutation can be reprogrammed into iPSCs. The Hb-CS mutation is repaired after introduction of the gRNA and single base editor for repairing the Hb-CS mutation, and the cells are induced to differentiate into hematopoietic stem/progenitor cells or erythroid progenitor cells.

The present disclosure also discloses a reagent for repairing an HBA2 gene mutation, comprising:
1) the single base editor protein or protein composition as described above, or a nucleotide sequence encoding the single base editor;
   and
2) the gRNA as described above, or a nucleotide sequence encoding the gRNA, or a nucleotide composition comprising the nucleotide sequence encoding the gRNA.

It can be understood that the single base editor protein refers to an active protein with single base editing capabilities; the protein composition refers to a protein composition with single base editing capabilities, such as a protein composition comprising an active protein with a target nucleic acid-binding structural unit and an active protein with deaminase structural unit activity; the nucleotide composition may be a mixture of DNA and RNA.

The reagent for repairing an HBA2 gene mutation, single base editor, and/or gRNA thereof can be delivered to host cells *in vitro, ex vivo,* or *in vivo* via plasmid vectors, viral vectors, ribonucleoprotein complexes, virus-like vectors, *etc.,* and can function normally and provide a therapeutic effect by repairing the HBA2 gene.

The present disclosure also discloses a delivery system for repairing an HBA2 gene mutation, which is used to deliver the reagent as described above.

It can be understood that for the delivery system, conventional delivery systems in the art, such as liposome delivery, lipid nanoparticle delivery, extracellular vesicles (EVs), viral particles, or electroporation, can be used to deliver the reagent into the cells.

Compared to the prior art, the present disclosure has the following beneficial effects:
The present disclosure provides a method for repairing an HBA2 gene mutation by single base editing, which uses single base editing without generating double-strand breaks and affecting chromatin conformation and genome stability compared to conventional gene editing; without generating random insertions of large-fragment genes into genomes, which have a low impact on oncogene activation or tumor suppressor gene inactivation; and without generating random insertions/deletions at other sites in a genome, causing unpredictable risks. Moreover, the expression of a repaired target gene is regulated by all regulatory elements of natural HBA2, which is safe and more capable of balancing the expression of α/β globin, resulting in a better therapeutic effect.

Moreover, the gRNA obtained by further screening in the present disclosure has high repair efficiency, especially the gRNA with the Hb-CS mutation site at C13 to C17, which also has the advantages of low off-target editing probability and high safety. Furthermore, the gRNA with minimal PAM site restriction is obtained by screening in the present disclosure, especially the gRNA with the Hb-CS mutation site at C16, which is almost unaffected by PAM site limitation.

### BRIEF DESCRIPTION OF THE DRAWINGS

FIG. 1 shows a schematic diagram of the structure of the pLenti6-GFP-HbCS reporter vector in Example 1;
FIG. 2 shows a graph of sequencing and efficiency analysis of the base editing results at the Hb-CS site in Example 4;
FIG. 3 shows a schematic diagram of the efficiency of repairing an Hb-CS mutation using gRNAs with a guide sequence length of 15 to 25 nt targeting the Hb-CS mutation at C16 in combination with a single base editor in Example 5;
FIG. 4 shows a schematic diagram of the efficiency of repairing an Hb-CS mutation using gRNAs with a guide sequence length of 15 to 25 nt targeting the Hb-CS mutation at C15 in combination with a single base editor in Example 5;
FIG. 5 shows a schematic diagram of the efficiency of repairing an Hb-CS mutation using gRNAs with a guide sequence length of 15 to 25 nt targeting the Hb-CS mutation at C17 in combination with a single base editor in Example 5;
FIG. 6 shows a graph of sequencing and efficiency analysis of the results of repairing an Hb-CS site mutation using the AncBE single base editor in Example 6;
FIG. 7 shows a graph of sequencing and efficiency analysis of the results of repairing an Hb-CS mutation using the YE1-CBE single base editor in Example 7;
FIG. 8 shows a schematic diagram of the gRNA guide sequence mismatch in Example 8, in which the shaded bases represent the mismatched bases and the underlined bases represent the Hb-CS mutation site.

### DETAILED DESCRIPTION OF THE PREFERRED EMBODIMENT

To facilitate the understanding of the present disclosure, the present disclosure will be described more comprehensively below with reference to the relevant drawings. Preferred examples of the present disclosure are given in the drawings. However, the present disclosure may be implemented in many different forms and is not limited to the examples described herein. On the contrary, the purpose of providing these examples is to enable a more thorough and comprehensive understanding of the disclosure of the present disclosure.

Unless otherwise defined, all technical and scientific terms used herein have the same meaning as commonly understood by those skilled in the art. The terms used herein in the specification of the present disclosure are only for the purpose of describing specific examples and are not intended to limit the present disclosure. The term "and/or" as used herein comprises any and all combinations of one or more of the related listed items.

### Definitions:

Variant: referring to a group of highly similar protein members that are homologous to a single protein or protein family and have the same or similar biological functions. A variant of the CRISPR/Cas nuclease of the present disclosure refers to a homologous protein of CRISPR/Cas, which is a nucleic acid-binding protein programmed to target any desired nucleotide sequence within the genome. The deaminase variant of the present disclosure refers to a homologous protein of deaminase with base deamination. For example, a protein comprising the CRISPR/Cas nuclease or fragment thereof may be referred to as a "CRISPR/Cas nuclease variant", such as a Cas9 variant. The CRISPR/Cas nuclease variant shares homology with the CRISPR/Cas nuclease or fragment thereof. For example, the CRISPR/Cas nuclease variant is at least about 70% identical, at least about 80% identical, at least about 90% identical, at least about 95% identical, at least about 96% identical, at least about 97% identical, at least about 98% identical, at least about 99% identical, at least about 99.5% identical, or at least about 99.9% identical to the wild-type CRISPR/Cas nuclease.

In some embodiments, the CRISPR/Cas nuclease variant, compared to the wild-type CRISPR/Cas nuclease, may have 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more amino acid changes, but still retains the function of binding to programmable nucleotides such as gRNA and targeting nucleic acid targets.

Chemically synthesized modified gRNA refers to a gRNA that is modified using chemical modification methods well known to those skilled in the art, such as in some embodiments, 2'-O-methyl (2'-O-Me) modification or 2'-fluoro (2'-F) modification. In some embodiments, the modification between nucleotides of the gRNA comprises a phosphorothioate (PS) bond. In some embodiments, the first three nucleotides at the 5' end and the last three nucleotides at the 3' end are modified. In some embodiments, the first four nucleotides at the 5' end and the last four nucleotides at the 3' end are linked to a phosphorothioate (PS) bond.

In some embodiments, the gRNA may also use modified ribonucleotides, deoxyribonucleotides, other synthetic bases, and synthetic backbone linkages (such as peptide nucleic acid (PNA) and locked nucleic acid (LNA)). In some embodiments, a nucleic acid comprising the guide nucleotide sequence comprises natural nucleotides (e.g., adenosine, thymidine, guanosine, cytidine, uridine, deoxyadenosine, deoxythymidine, deoxyguanosine, and deoxycytidine); nucleotide analogs (*e.g.,* 2-aminoadenosine, 2-thiothymidine, inosine, pyrrolopyrimidine, 3-methyladenosine, 5-methylcytidine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (e.g., methylated bases); intercalated bases; modified sugars (e.g., 2'-fluororibose, ribose, 2'-deoxyribose, arabinose, and hexose); and/or modified phosphate groups (e.g., phosphorothioate and 5'-N-phosphoramidite linkages).

Unless otherwise specified, the reagents used in the following examples are commercially available; and unless otherwise specified, the methods used in the following examples can be implemented by conventional methods.

### Example 1

Construction of Hb-CS mutation-GFP reporter vector and cells.
1. Design and construction of GFP-HbCS reporter vector

The HBA2 gene fragment containing the Hb-CS mutation (SEQ ID NO: 63), TTCTGTGAGCACCGTGCTGACCTCCAAATACCGTCAAGCTGGAGCCTCGGTAGCC GTTCCTCCTGCCCGCTGGGCCTCCCAACGGGCCCTCCTCCCCTCCTTGCACCGGCC CTTCCTGGTCTTTGAATAAAGTCTGAGTGGGCAGCAGCCTGTGTGTGCCTGGGTTC TCTCTATCCCGGAATGTGCCAACAATGGAGGTGTTTACCTGTCTCAGACCAAGGAC CTCTCTGCAGCTGCATGGGGCTGGGGAGGGAGAACTGCAGGGAGTATGGGAGGG GAAGCTGAGGTG, was selected.

In the above sequence fragment, the capital C in bold and underlined represents the Hb-CS (Hb Constant Spring) mutation, *i.e.,* HBA2: c.427T>C. The stop codon TAA of GFP was replaced and GFP gene lacks a stop codon to emit green fluorescence.

Synthesis of target gene fragment: The following gene fragment was designed: CMV promoter + GFP gene (with the stop codon TAA removed) + HBA2 gene fragment (Hb-CS mutation). The target fragment was inserted into a lentiviral backbone vector to construct a lentiviral vector containing pCMV-GFP-HBA2 (HbCS), and the sequence was verified by sequencing. Positive clones were used for lentiviral packaging.

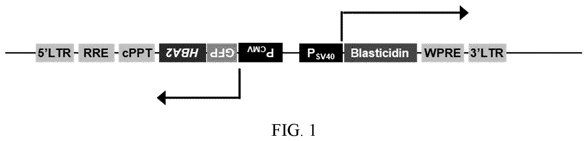

Specifically, the pLenti6/V5-GW/lacZ vector (Invitrogen, Cat. No. V49610 kit) was used as a lentiviral backbone vector in this example. The target gene fragment was inserted in reverse orientation to replace the CMV promoter and lacZ gene fragment of the original backbone vector. The structure of the pLenti6-GFP-HbCS reporter vector is shown in FIG. 1, which comprises a lentiviral backbone, an antibiotic module containing the promoter PSV40-Blasticidin, and a target gene module containing the promoter pCMV-GFP (without stop codon)-HBA2 fragment (Hb-CS mutation).

### 2. Packaging and concentration of lentivirus

293T cells were thawed and cultured to the exponential growth phase, and then transfected with lentiviral plasmid and helper plasmid using Lipofectamine 2000. After 48 to 72 hours, the cells were recovered and the virus was concentrated. The 293T cells were inoculated and proliferated until 80 to 90% confluence was reached for later use in lentiviral packaging.

One hour before transfection, the medium was replaced with fresh complete medium. The specific steps to prepare a transfection complex were illustrated using the transfection in a T25 flask as an example:
(i) Plasmids were added to 500 µL of OPTI-MEM and mixed well. The plasmid amounts were as follows: pCMV-VSV-G (source: ADDGENE #8454): pMDLg (source: ADDGENE #12251): pRSV-Rev (source: ADDGENE #12253): pLenti6-GFP-HbCS = 2 µg: 4 µg: 3 µg: 5 to 6 µg.
(ii) Lipo2000, three times the amount of plasmid, was added to 500 µL of OPTI-MEM and mixed well.

The mixtures in (i) and (ii) were placed at room temperature for 5 minutes, mixed well, and placed for another 20 minutes to prepare a transfection complex.

The transfection complex was evenly added to a cell culture flask, followed by incubation for 4 to 6 hours. The medium was replaced with fresh complete medium, followed by incubation for 48 to 72 hours. The culture supernatant was collected and centrifuged at 1500 g for 10 minutes, and the supernatant was collected again. The supernatant was filtered through a 0.45 µM PVDF syringe filter. 1/5 volume of 50% PEG 6000 was added and mixed well, and the mixture was placed at 4°C overnight. A small amount of precipitate was visible. The mixture was centrifuged at 1500 g for 10 minutes to collect the precipitate at the bottom of the tube, and the supernatant was discarded. The pellet was resuspended in serum-free DMEM to an appropriate volume, resulting in the concentrated GFP-HbCS lentivirus.

### 3. Construction of GFP-HbCS reporter cell model

Host cells to be transfected (293T cells, K562 cells, human HUDEP2 cells, human hematopoietic stem cells, erythroid progenitor/precursor cells, *etc*.) were inoculated into a 6-well plate. 5 µL to 10 µL of concentrated GFP-HbCS lentivirus was added to each well. The mixture was added with Blasticidin antibiotic (at a concentration of 10 to 40 µg/mL) and incubated for 48 to 72 hours. PCR was used to identify whether the positive fragment was inserted.

Cells with positive clones were dispensed into a 96-well cell culture plate. Positive monoclonal cell lines were screened out and stored as a GFP-HbCS reporter cell model for later use.

### Example 2

Design and synthesis of gRNA.

In order to realize the editing and repairing of the target base, in the present disclosure, gRNA (guide RNA) was used to achieve the effect of guiding and localization. The guide sequence of the gRNA binds to the target nucleic acid region, while the scaffold sequence of the gRNA binds to the target nucleic acid-binding structural unit of the single base editor, thus guiding the editing and repairing of the target base by the single base editor. In this example, gRNA was designed and synthesized.

### 1. Screening of gRNA

The gRNA sequence comprises a guide sequence that binds to or is reverse complementary to the region Chr16: 173548-173648 within 50 nucleotides upstream and downstream of the Hb-CS mutation site to be edited or the nucleotide sequence (ccgttgggaggcccagcgggcaggaggaacggctaccgaggctccagcttAacggtatttggaggtcagcacggtgctcacaga agccaggaacttgtcca) (SEQ ID NO: 64), as well as a scaffold sequence that binds to the single base editor.

In the present disclosure, the design of the gRNA guide sequence is based on the editing window requirements of the single base editor and other principles such as 12 to 18 nucleotides from the PAM or 3' end of the guide sequence, and the length of the gRNA guide sequence of at least 16 bp are taken into consideration together.

Meanwhile, due to the high homology between the HBA2 and HBA1 genes, HBA2-specific target sites or target sequences covering the Hb-CS mutation should be preferred in the process of guide sequence design.

By combining these factors for sequence analysis and screening, 62 gRNA guide sequences were selected in the present disclosure (as shown in Table 1).

**Table 1. List of gRNA guide sequences, gRNA binding sites/target sequences, and Hb-CS mutation localization**

| gRNA No. | SEQ ID NO: | DNA nucleotide sequence encoding guide sequence (5'-3') | Binding site localization (antisense strand) | Hb-CS mutation localization | Guide sequence length (nt) |
|---|---|---|---|---|---|
| 1 | 1 | CCGTCAAGCTGGAGCCTCG G | Chr16: 173594-173613 | C16 | 20 |
| 2 | 2 | AAGCTGGAGCCTCGG | Chr16: 173599-173613 | C16 | 15 |
| 3 | 3 | CAAGCTGGAGCCTCGG | Chr16: 173598-173613 | C16 | 16 |
| 4 | 4 | TCAAGCTGGAGCCTCGG | Chr16: 173597-173613 | C16 | 17 |
| 5 | 5 | GTCAAGCTGGAGCCTCGG | Chr16: 173596-173613 | C16 | 18 |
| 6 | 6 | CGTCAAGCTGGAGCCTCGG | Chr16: 173595-173613 | C16 | 19 |
| 7 | 7 | ACCGTCAAGCTGGAGCCTC GG | Chr16: 173593-173613 | C16 | 21 |
| 8 | 8 | TACCGTCAAGCTGGAGCCT CGG | Chr16: 173592-173613 | C16 | 22 |
| 9 | 9 | ATACCGTCAAGCTGGAGCC TCGG | Chr16: 173591-173613 | C16 | 23 |
| 10 | 10 | AATACCGTCAAGCTGGAGC CTCGG | Chr16: 173590-173613 | C16 | 24 |
| 11 | 11 | AAATACCGTCAAGCTGGAG CCTCGG | Chr16: 173589-173613 | C16 | 25 |
| 12 | 12 | CGTCAAGCAGGAGCCTCGG | Chr16: 173595-173613 | C16 | 19 |
| 13 | 13 | TCTCAAGCTGGAGCCTCGG | Chr16: 173595-173613 | C16 | 19 |
| 14 | 14 | CGTCACTGAGGAGCCTCGG | Chr16: 173595-173613 | C16 | 19 |
| 15 | 15 | ACCGTCAAGCTGGAGCCTC G | Chr16: 173593-173612 | C15 | 20 |
| 16 | 16 | CAAGCTGGAGCCTCG | Chr16: 173598-173612 | C15 | 15 |
| 17 | 17 | TCAAGCTGGAGCCTCG | Chr16: 173597-173612 | C15 | 16 |
| 18 | 18 | GTCAAGCTGGAGCCTCG | Chr16: 173596-173612 | C15 | 17 |
| 19 | 19 | CGTCAAGCTGGAGCCTCG | Chr16: 173595-173612 | C15 | 18 |
| 20 | 20 | CCGTCAAGCTGGAGCCTCG | Chr16: 173594-173612 | C15 | 19 |
| 21 | 21 | TACCGTCAAGCTGGAGCCT CG | Chr16: 173592-173612 | C15 | 21 |
| 22 | 22 | ATACCGTCAAGCTGGAGCC TCG | Chr16: 173591-173612 | C15 | 22 |
| 23 | 23 | AATACCGTCAAGCTGGAGC CTCG | Chr16: 173590-173612 | C15 | 23 |
| 24 | 24 | AAATACCGTCAAGCTGGAG CCTCG | Chr16: 173589-173612 | C15 | 24 |
| 25 | 25 | CAAATACCGTCAAGCTGGA GCCTCG | Chr16: 173588-173612 | C15 | 25 |
| 26 | 26 | ATACCGTCAAGCTGGAGCC T | Chr16: 173591-173610 | C13 | 20 |
| 27 | 27 | GTCAAGCTGGAGCCT | Chr16: 173596-173610 | C13 | 15 |
| 28 | 28 | CGTCAAGCTGGAGCCT | Chr16: 173595-173610 | C13 | 16 |
| 29 | 29 | CCGTCAAGCTGGAGCCT | Chr16: 173594-173610 | C13 | 17 |
| 30 | 30 | ACCGTCAAGCTGGAGCCT | Chr16: 173593-173610 | C13 | 18 |
| 31 | 31 | TACCGTCAAGCTGGAGCCT | Chr16: 173592-173610 | C13 | 19 |
| 32 | 32 | AATACCGTCAAGCTGGAGC CT | Chr16: 173590-173610 | C13 | 21 |
| 33 | 33 | AAATACCGTCAAGCTGGAG CCT | Chr16: 173589-173610 | C13 | 22 |
| 34 | 34 | CAAATACCGTCAAGCTGGA GCCT | Chr16: 173588-173610 | C13 | 23 |
| 35 | 35 | CCAAATACCGTCAAGCTGG AGCCT | Chr16: 173587-173610 | C13 | 24 |
| 36 | 36 | TCCAAATACCGTCAAGCTG GAGCCT | Chr16: 173586-173610 | C13 | 25 |
| 37 | 37 | CGTCAAGCTGGAGCCTCGG T | Chr16: 173595-173614 | C17 | 20 |
| 38 | 38 | AGCTGGAGCCTCGGT | Chr16: 173600-173614 | C17 | 15 |
| 39 | 39 | AAGCTGGAGCCTCGGT | Chr16: 173599-173614 | C17 | 16 |
| 40 | 40 | CAAGCTGGAGCCTCGGT | Chr16: 173598-173614 | C17 | 17 |
| 41 | 41 | TCAAGCTGGAGCCTCGGT | Chr16: 173597-173614 | C17 | 18 |
| 42 | 42 | GTCAAGCTGGAGCCTCGGT | Chr16: 173596-173614 | C17 | 19 |
| 43 | 43 | CCGTCAAGCTGGAGCCTCG GT | Chr16: 173594-173614 | C17 | 21 |
| 44 | 44 | ACCGTCAAGCTGGAGCCTC GGT | Chr16: 173593-173614 | C17 | 22 |
| 45 | 45 | TACCGTCAAGCTGGAGCCT CGGT | Chr16: 173592-173614 | C17 | 23 |
| 46 | 46 | ATACCGTCAAGCTGGAGCC TCGGT | Chr16: 173591-173614 | C17 | 24 |
| 47 | 47 | AATACCGTCAAGCTGGAGC CTCGGT | Chr16: 173590-173614 | C17 | 25 |
| 48 | 48 | TACCGTCAAGCTGGAGCCT C | Chr16: 173592-173611 | C14 | 20 |
| 49 | 49 | TCAAGCTGGAGCCTC | Chr16: 173596-173611 | C14 | 15 |
| 50 | 50 | GTCAAGCTGGAGCCTC | Chr16: 173596-173611 | C14 | 16 |
| 51 | 51 | CGTCAAGCTGGAGCCTC | Chr16: 173595-173611 | C14 | 17 |
| 52 | 52 | CCGTCAAGCTGGAGCCTC | Chr16: 173594-173611 | C14 | 18 |
| 53 | 53 | ACCGTCAAGCTGGAGCCTC | Chr16: 173593-173611 | C14 | 19 |
| 54 | 54 | ATACCGTCAAGCTGGAGCC TC | Chr16: 173591-173611 | C14 | 21 |
| 55 | 55 | AATACCGTCAAGCTGGAGC CTC | Chr16: 173590-173611 | C14 | 22 |
| 56 | 56 | AAATACCGTCAAGCTGGAG CCTC | Chr16: 173589-173611 | C14 | 23 |
| 57 | 57 | CAAATACCGTCAAGCTGGA GCCTC | Chr16: 173588-173611 | C14 | 24 |
| 58 | 58 | CCAAATACCGTCAAGCTGG AGCCTC | Chr16: 173587-173611 | C14 | 25 |
| 59 | 59 | AATACCGTCAAGCTGGAGC C | Chr16: 173590-173609 | C12 | 20 |
| 60 | 60 | CCGTCAAGCTGGAGCC | Chr16: 173594-173609 | C12 | 16 |
| 61 | 61 | GTCAAGCTGGAGCCTCGGT A | Chr16: 173596-173615 | C18 | 20 |
| 62 | 62 | AGCTGGAGCCTCGGTA | Chr16: 173600-173615 | C18 | 16 |

The "Hb-CS mutation localization" above refers to the localization of the Hb-CS mutation site in the single base editor-gRNA, *i.e.,* the distance between the 3' end of the gRNA guide sequence and the codon cytosine at CD142 of the HBA2 gene sequence, and specifically, in this example, is represented by the position of the Hb-CS mutation base in the gRNA guide sequence, *i.e.,* the x-th nucleotide from the 3' end of the guide sequence, or the x-th nucleotide from the PAM site (in cases where a PAM site is present), abbreviated as Cx.

It can be understood that the gRNA can be selected from a dual-molecule guide RNA (dgRNA, *i.e.,* crRNA and trRNA), a single-molecule guide RNA (sgRNA), or a multi-molecule guide RNA.

In this example, a single-molecule gRNA was used, which contains the following structure: 5'-[guide sequence]-guuuuagagcuagaaauagcaaguuaaaauaaggcuaguccguuaucaacuugaaaaaguggcaccgagu cggugcuuuu[gRNA scaffold sequence] (SEQ ID NO: 65)-3'. For example, if the gRNA guide sequence corresponds to the No. 1 target sequence above, the complete gRNA sequence is

ccgucaagcuggagccucggguuuuagagcuagaaauagcaaguuaaaauaaggcuaguccguuaucaacuugaaaaag
uggcaccgagucggugcuuuu (SEQ ID NO: 66).

### 2. Synthesis of gRNA

### 2.1 Construction of gRNA plasmid

Using conventional methods, the sense and antisense strands of the DNA sequence corresponding to the gRNA guide sequence in Table 1 above were synthesized, with the addition of "caccg" to the 5'-end of the sense strand, "aaac" to the 5'-end of the antisense strand, and "C" to the 3'-end of the antisense strand.

2 µL of the sense strand (Oligo-F) and antisense strand (Oligo-R) of the DNA sequence respectively corresponding to the above gRNA guide sequence were mixed, followed by addition of 2 µL of NEB 10x CutSmart buffer and 14 µL of H₂O. The mixture was incubated at 95°C for 5 minutes in a PCR machine, then immediately removed and incubated on ice for 5 minutes, and annealed to form a double-stranded guide sequence DNA with sticky ends.

The double-stranded guide sequence DNA with sticky ends was ligated to the BpiI-digested product (linearized vector) of the plasmid vector containing the gRNA scaffold sequence (e.g., PUC19-U6 promoter-BpiI-BpiI-gRNA scaffold; the PUC19-U6 background plasmid was derived from ADDGENE #121958, with the gRNA scaffold sequence: gttttagagctagaaatagcaagttaaaataaggctagtccgttatcaacttgaaaaagtggcaccgagtcggtgc (SEQ ID NO: 67)) by T4 ligase. The ligation product was transformed, and monoclonal colonies were selected for PCR testing and Sanger sequencing, followed by screening to obtain positive clones, *i.e.,* gRNA plasmid.

### 2.2. Synthesis and chemical modification of gRNA

Based on the list of gRNA guide sequences in Table 1 above, methyl and phosphorothioate bond-modified gRNAs were obtained through chemical synthesis and modification for later use.

For example, the synthesized and modified single-molecule gRNA-1 has the following synthetic sequence and modification: 5'-mC*mC*mG*UCAAGCUGGAGCCUCGG-GUUUUAGAGCUAGAAAUAGCAAGUU AAAAUAAGGCUAGUCCGUUAUCAACUUGAAAAAGUGGCACCGAGUCGGUGCU* mU*mU*mU-3' (underlined part represents the guide sequence, m represents 2'-O-methyl modification, and * represents phosphorothioate bond modification).

### Example 3

Single base editor and gRNA were delivered to host cells.

### 1. Cell thawing

The GFP-HbCS reporter cell model successfully constructed in step 3 of Example 1 was thawed, and the cells were plated into a 6-well cell culture plate. The plate was incubated for 24 hours in a CO₂ incubator for cell transfection.

### 2. Liposome transfection

Host cells at the logarithmic growth phase cultured in the 6-well plate were washed with 1 mL of PBS, and digested or recovered to obtain a host cell resuspension/suspension solution, 20 µL of which was taken for cell counting. The cells were plated into a 24-well plate at a density of 2 × 10⁵ cells/well. Each well was supplemented with medium to a volume of approximately 500 µL.

### 3. Preparation of transfection complex A

OPTI-MEM obtained in Example 1, gRNA plasmid obtained in Example 2, and base editor plasmid were sequentially added to a 1.5 mL EP tube, and gently mixed. The mixture was incubated, and allowed to stand at room temperature for 20 minutes to form a complex, followed by addition of 40 µL of OPTI-MEM.

### 4. Preparation of transfection complex B

OPTI-MEM and Lipofectamine 2000 transfection reagent were sequentially added to a 1.5 mL EP tube. The mixture was gently mixed and allowed to stand at room temperature for 10 minutes. Transfection complex A and transfection complex B were gently mixed and allowed to stand at room temperature for 15 minutes. The mixture was then added to a 24-well plate at 50 µL per well and incubated for 72 hours.

### 5. Plasmid electroporation

The host cells were transferred from the 6-well plate to a T25 culture flask and incubated for 24 hours, during which the cell density was carefully controlled. The cell culture medium was transferred from the T25 flask to a 15 mL centrifuge tube and centrifuged at 90 × g for 10 minutes. The supernatant was discarded, and the cells were resuspended in 1 mL of PBS. Cell counting was performed. 0.5 × 10⁶ cells were taken and centrifuged at 90 × g for 10 minutes. The supernatant was discarded, and the cells were resuspended in approximately 20 µL of electroporation mixture (a mixture of single base editor plasmid solution (a plasmid mass of 300 ng to 5 µg)/gRNA plasmid solution (a plasmid mass of 300 ng to 5 µg)/SF-P3 solution/Supplement solution, following the instructions provided by Lonza (Cat. No. V4XC-2032)). The mixture was transferred to an electroporation cuvette and subjected to electroporation by selecting the electroporation program corresponding to the host cells.

After electroporation, the mixture was allowed to stand at room temperature for 10 minutes, then added with 80 µL of RPMI 1640 medium (containing 10% FBS), and gently pipetted 3 times. The cells were transferred to a 24-well plate for incubation, and each well was supplemented with medium to a volume of 400 µL.

### 7. RNA system electroporation

Single base editor mRNA and chemically synthesized gRNA were delivered to host cells by electroporation. The host cells were transferred from the 6-well plate to a T25 culture flask and incubated for 24 hours, during which the cell density was carefully controlled. The cell culture medium was transferred from the T25 flask to a 15 mL centrifuge tube and centrifuged at 90 × g for 10 minutes. The supernatant was discarded, and the cells were resuspended in 1 mL of PBS. Cell counting was performed. 0.5 × 10⁶ cells were taken and centrifuged at 90 × g for 10 minutes. The supernatant was discarded, and the cells were resuspended in approximately 20 µL of electroporation mixture (a mixture of single base editor mRNA solution/gRNA solution/SF-P3 solution/Supplement solution, following the instructions provided by Lonza (Cat. No. V4XC-2032)). The mixture was transferred to an electroporation cuvette and subjected to electroporation by selecting the electroporation program corresponding to the host cells.

After electroporation, the mixture was allowed to stand at room temperature for 10 minutes, then added with 80 µL of RPMI 1640 medium (containing 10% FBS), and gently pipetted 3 times. The cells were transferred to a 24-well plate for incubation, and each well was supplemented with medium to a volume of 400 µL.

### 8. RNP electroporation

Single base editor protein and chemically synthesized gRNA were packaged into ribonucleoprotein (RNP) through *in vitro* incubation. RNP was delivered to host cells by electroporation. The host cells were transferred from the 6-well plate to a T25 culture flask and incubated for 24 hours, during which the cell density was carefully controlled. The cell culture medium was transferred from the T25 flask to a 15 mL centrifuge tube and centrifuged at 90 × g for 10 minutes. The supernatant was discarded, and the cells were resuspended in 1 mL of PBS. Cell counting was performed. 0.5 × 10⁶ cells were taken and centrifuged at 90 × g for 10 minutes. The supernatant was discarded, and the cells were resuspended in approximately 20 µL of electroporation mixture (a mixture of single base editor protein solution/gRNA solution/SF-P3 solution/Supplement solution, following the instructions provided by Lonza (Cat. No. V4XC-2032)). The mixture was transferred to an electroporation cuvette and subjected to electroporation by selecting the electroporation program corresponding to the host cells.

After electroporation, the mixture was allowed to stand at room temperature for 10 minutes, then added with 80 µL of RPMI 1640 medium (containing 10% FBS), and gently pipetted 3 times. The cells were transferred to a 24-well plate for incubation, and each well was supplemented with medium to a volume of 400 µL.

### 9. Lipid nanoparticle (LNP) delivery

Plasmid DNA, mRNA, or ribonucleoprotein complexes of the single base editor and gRNA were encapsulated in "stabilized plasmid-lipid particles (SPLP)" containing the fusion lipid dioleoylphosphatidylethanolamine (DOPE) and low levels (5 to 10 mol%) of cationic lipid, and stabilized by a polyethylene glycol (PEG) coating. LNP delivery can be used for *in vitro* delivery of single base editor-gRNA into cells and can also be used for delivery in organisms.

### 10. Post-transfection sequencing

After the incubation of transfected cells for 72 hours, the host cells co-transfected with the single base editor plasmid and gRNA plasmid were recovered. The genes were extracted, and the target fragment was amplified by PCR. The PCR primer sequences were as follows: (forward primer) CGACAAGCAGAAGAACGGCATCAA (SEQ ID NO: 68), (reverse primer) CAGCTGCAGAGAGGTCCTTG (SEQ ID NO: 69). The PCR product was subjected to Sanger sequencing.

### 11. Result analysis

The sequencing results were analyzed for gene editing efficiency using the EditR 10.10 website (https://moriaritylab.shinyapps.io/editr_v10/).

### Example 4

Repair of Hb-CS mutation by gRNA-guided single base editor.

### 1. Method

In this example, referring to the method of plasmid electroporation in Example 3, gRNA-1 plasmid, gRNA-15 plasmid, gRNA-26 plasmid, gRNA-37 plasmid, gRNA-48 plasmid, gRNA-59 plasmid, and gRNA-61 plasmid targeting the Hb-CS mutation at C12 to 18 were respectively mixed with pCAG-CBE4max-SpRY-P2A-EGFP (source: Addgene Plasmid #139999) and electroporated into K562 cell lines. After the incubation of transfected cells for 72 hours, the host cells co-transfected with the single base editor plasmid and gRNA plasmid were recovered. The genes were extracted, and the target fragment was amplified by PCR. The PCR product was subjected to Sanger sequencing.

### 2. Results

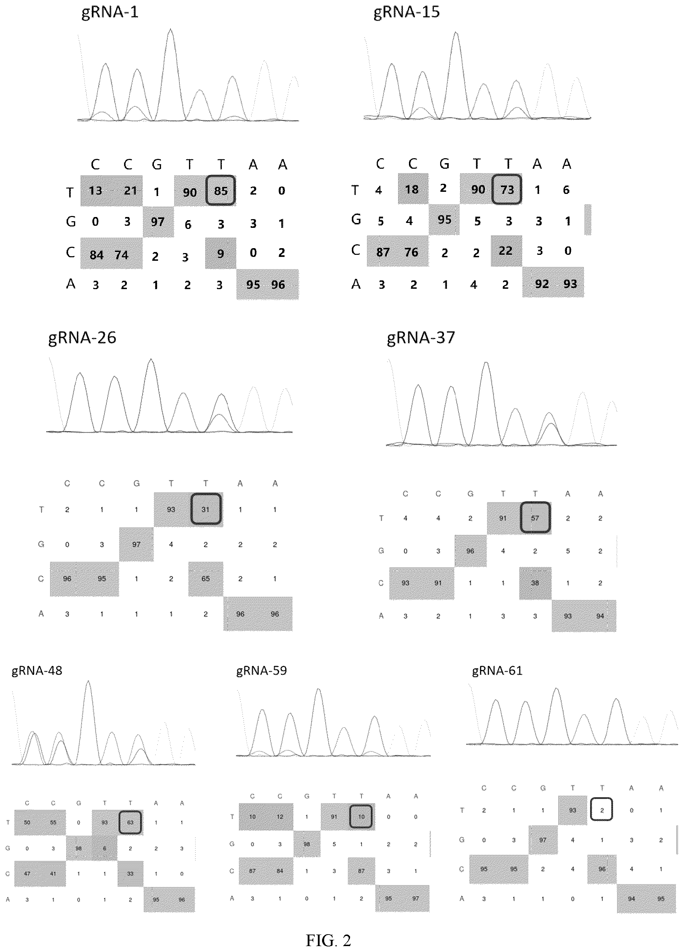

The sequencing results were analyzed for gene editing efficiency using the EditR 10.10 website, as shown in the table below and FIG. 2. FIG. 2 shows a graph of sequencing and efficiency analysis of base editing results at the Hb-CS site, in which the numbers in the boxed areas represent the efficiency (%) of target base editing.

**Table 2. Repair of Hb-CS mutation by SpRY single base editor**

| Experimental group | Proportion of Hb-CS mutations repaired | |
|---|---|---|
| | Parallel experiment 1 | Parallel experiment 2 |
| gRNA-1 | 85% | 82% |
| gRNA-15 | 86% | 73% |
| gRNA-26 | 24% | 31% |
| gRNA-37 | 65% | 57% |
| gRNA-48 | 64% | 63% |
| gRNA-59 | 10% | 10% |
| gRNA-61 | 2% | 2% |

The above results show that the SpRY single base editor mediated by gRNA-1 (C16), gRNA-15 (C15), gRNA-26 (C13), gRNA-37 (C17), gRNA-48 (C14), and gRNA-59 (C12) can repair the Hb-CS mutation of the HBA2 gene. Moreover, the repair efficiency of gRNA-1, gRNA-15, gRNA-26, gRNA-37, and gRNA-48 at the Hb-CS mutation site is significantly higher than that of gRNA-59.

**Table 3. Non-target editing efficiency of SpRY single base editor in repairing Hb-CS mutation**

| Experimental group | Non-target site 1 editing efficiency (3rd base at the 5' end of the Hb-CS site) | | Non-target site 2 editing efficiency (4th base at the 5' end of the Hb-CS site) | |
|---|---|---|---|---|
| | Parallel experiment 1 | Parallel experiment 2 | Parallel experiment 1 | Parallel experiment 2 |
| gRNA-1 | 21% | 23% | 13% | 14% |
| gRNA-15 | 27% | 18% | 7% | 4% |
| gRNA-26 | 5% | 1% | 5% | 2% |
| gRNA-37 | 4% | 4% | 5% | 4% |

The above results show that, compared to gRNA-1, gRNA-15, gRNA-26, gRNA-37, and gRNA-48, the non-target editing efficiency of gRNA-59 is very high and is higher than the editing efficiency at the target site. The on-target editing efficiency of gRNA-1, gRNA-15, gRNA-26, gRNA-37, and gRNA-48 is higher than their non-target editing efficiency, while gRNA-1, gRNA-15, gRNA-26, and gRNA-37 exhibit lower off-target editing efficiency.

### Example 5

Experiment on repairing Hb-CS mutation using single base editors mediated by gRNAs of different lengths.

In this example, referring to the method of plasmid electroporation in Example 3, gRNAs of different guide sequence lengths with the same Hb-CS mutation localization (number of bases measured from the 3' end of the guide sequence) were co-delivered with the CBE single base editor into host cells, so as to observe whether gRNAs of different lengths can induce the repair of the Hb-CS mutation site, and to test the effect of different gRNA lengths on repair efficiency.

### 1. Method

### 1.1 Electroporation

1) gRNAs of different guide sequence lengths targeting the Hb-CS mutation at C16 (measured from the 3' end of the guide sequence), including gRNA-2, gRNA-3, gRNA-4, gRNA-6, and gRNA-11, were respectively mixed with pCAG-CBE4max-SpRY-P2A-EGFP (source: Addgene Plasmid #139999) and electroporated into K562 cell lines.
2) gRNAs of different guide sequence lengths targeting the Hb-CS mutation at C15 (measured from the 3' end of the guide sequence), including gRNA-16, gRNA-17, gRNA-18, gRNA-20, and gRNA-25, were respectively mixed with pCAG-CBE4max-SpRY-P2A-EGFP (Addgene Plasmid #139999) and electroporated into K562 cell lines.
3) gRNAs of different guide sequence lengths targeting the Hb-CS mutation at C17 (measured from the 3' end of the guide sequence), including gRNA-38, gRNA-39, gRNA-40, gRNA-42, and gRNA-47, were respectively mixed with pCAG-CBE4max-SpRY-P2A-EGFP (Addgene Plasmid #139999) and electroporated into K562 cell lines.

### 1.2 Incubation

After the incubation of transfected cells for 72 hours, the host cells co-transfected with the single base editor plasmid and gRNA plasmid were recovered.

### 1.3 Sequencing

The genes were extracted, and the target fragment was amplified by PCR. The PCR product was subjected to Sanger sequencing.

### 2. Results

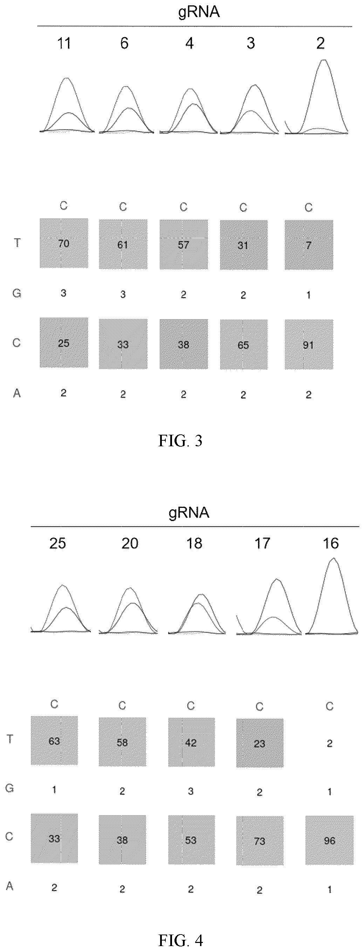

The sequencing results were analyzed for gene editing efficiency using the EditR 10.10 website, as shown in the table below and FIGs. 3 to 5.

**Table 4. Efficiency of repairing Hb-CS mutation using gRNAs with a guide sequence length of 15 to 25 nt targeting the Hb-CS mutation at C16 in combination with single base editor**

| gRNA | Guide sequence length (nt) | Hb-CS mutation localization (starts from the 3' end of the guide sequence) | Hb-CS repair efficiency |
|---|---|---|---|
| gRNA-11 | 25 | C16 | 70% |
| gRNA-6 | 19 | C16 | 61% |
| gRNA-4 | 17 | C16 | 57% |
| gRNA-3 | 16 | C16 | 31% |
| gRNA-2 | 15 | C16 | 7% |

**Table 5. Efficiency of repairing Hb-CS mutation using gRNAs with a guide sequence length of 15 to 25 nt targeting the Hb-CS mutation at C15 in combination with single base editor**

| gRNA | Guide sequence length (nt) | Hb-CS mutation localization (starts from the 3' end of the guide sequence) | Hb-CS repair efficiency |
|---|---|---|---|
| gRNA-25 | 25 | C15 | 63% |
| gRNA-20 | 19 | C15 | 58% |
| gRNA-18 | 17 | C15 | 42% |
| gRNA-17 | 16 | C15 | 23% |
| gRNA-16 | 15 | C15 | 2% |

**Table 6. Efficiency of repairing Hb-CS mutation using gRNAs with a guide sequence length of 15 to 25 nt targeting the Hb-CS mutation at C17 in combination with single base editor**

| gRNA | Guide sequence length (nt) | Hb-CS mutation localization (starts from the 3' end of the guide sequence) | Hb-CS repair efficiency |
|---|---|---|---|
| gRNA-47 | 25 | C17 | 44% |
| gRNA-42 | 19 | C17 | 34% |
| gRNA-40 | 17 | C17 | 23% |
| gRNA-39 | 16 | C17 | 16% |
| gRNA-38 | 15 | C17 | 3% |

The above experimental results show that gRNAs with a length of 16 to 25 nt targeting the Hb-CS mutation at C16, gRNAs with a length of 16 to 25 nt targeting the Hb-CS mutation at C15, and gRNAs with a length of 16 to 25 nt targeting the Hb-CS mutation at C17 respectively can repair the Hb-CS mutation in combination with a single base editor.

Moreover, based on the results of this example in conjunction with those of Examples 3 to 4, it can be known that gRNAs with a guide sequence length of 16 to 25 nt targeting the Hb-CS mutation at C13 to C17 (number of bases measured from the 3' end of the guide sequence) can each repair the Hb-CS mutation in combination with a single base editor.

Furthermore, gRNAs with a guide sequence length of 17 to 25 nt targeting the Hb-CS mutation at C13 to C17 (number of bases measured from the 3' end of the guide sequence) have a better Hb-CS mutation repair effect. Specifically, gRNAs that bind to or are reverse complementary to target nucleotide sequences of 16 bp or more starting at the positions chr16: 173613, 173612, 173610, 173614, and 173611 on the antisense strand of the human genome, can work together with a single base editor to induce the conversion of C to T at the Hb-CS mutation site of the HBA2 gene with good editing efficiency.

### Example 6

### Experiment on repairing Hb-CS mutation by AncBE single base editor

### 1. Method

In this example, referring to the method of plasmid electroporation in Example 3, gRNA-1, gRNA-26, gRNA-59, and gRNA-61 plasmids were respectively mixed with pCMV-AncBE4max-GFP (source: Addgene Plasmid #112100) and electroporated into K562 cell lines. After the incubation of transfected cells for 72 hours, the host cells co-transfected with the single base editor plasmid and gRNA plasmid were recovered. The genes were extracted, and the target fragment was amplified by PCR. The PCR product was subjected to Sanger sequencing.

### 2. Results

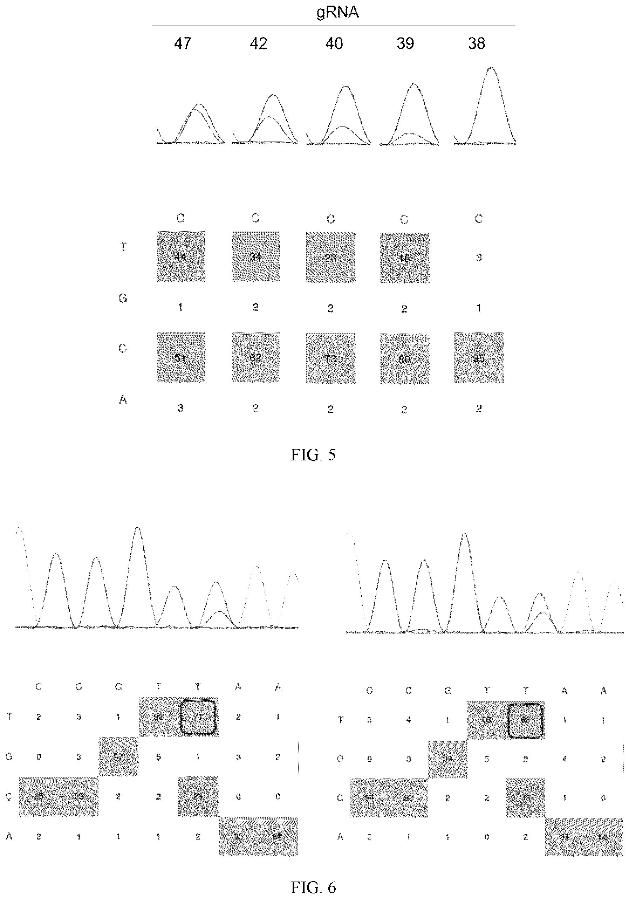

The sequencing results were analyzed for gene editing efficiency using the EditR 10.10 website, as shown in the table below and FIG. 6. FIG. 6 shows a graph of sequencing and efficiency analysis of the results of repairing the Hb-CS site mutation by the AncBE single base editor mediated by gRNA-1, in which the numbers in the boxed areas represent the efficiency (%) of on-target editing.

**Table 7. Repair of Hb-CS mutation by AncBE single base editor**

| Experimental group | Proportion of Hb-CS mutations repaired | |
|---|---|---|
| | Parallel experiment 1 | Parallel experiment 2 |
| gRNA-1 | 71% | 63% |
| gRNA-26 | 75% | 76% |
| gRNA-59 | 4% | 8% |
| gRNA-61 | 2% | 2% |

The above experimental results show that the AncBE single base editor mediated by gRNA-1 and gRNA-26 can repair the Hb-CS mutation of the HBA gene, with the repair efficiency significantly superior to that of gRNA-59 and gRNA-61. Additionally, experiments indicate that the off-target editing probability of gRNA-1 and gRNA-26 is lower than their on-target editing probability.

The AncBE single base editor mediated by gRNA-26 exhibits higher Hb-CS mutation repair efficiency compared to the combination with other single base editors. Although gRNA-1 is not an optimal PAM site for the nuclease in Anc-BE4, it unexpectedly demonstrates significant Hb-CS repair efficiency, which is much higher than the off-target editing efficiency by 2 to 4%. Moreover, the off-target editing efficiency of the AncBE single base editor guided by gRNA-1 is significantly lower than that of the SpRY single base editor. It can be seen that the PAM site is not a determining factor in limiting the Hb-CS repair efficiency by the single base editor.

### Example 7

### Experiment on repairing Hb-CS mutation by YE1-CBE single base editor

### 1. Method

In this example, referring to the method of plasmid electroporation in Example 3, gRNA-1, gRNA-15, gRNA-48, gRNA-59, and gRNA-61 plasmids were respectively mixed with YE1-BE4max-NG (source: Addgene Plasmid #138159) and electroporated into K562 cell lines. After the incubation of transfected cells for 72 hours, the host cells co-transfected with the single base editor plasmid and gRNA plasmid were recovered. The genes were extracted, and the target fragment was amplified by PCR. The PCR product was subjected to Sanger sequencing.

### 2. Results

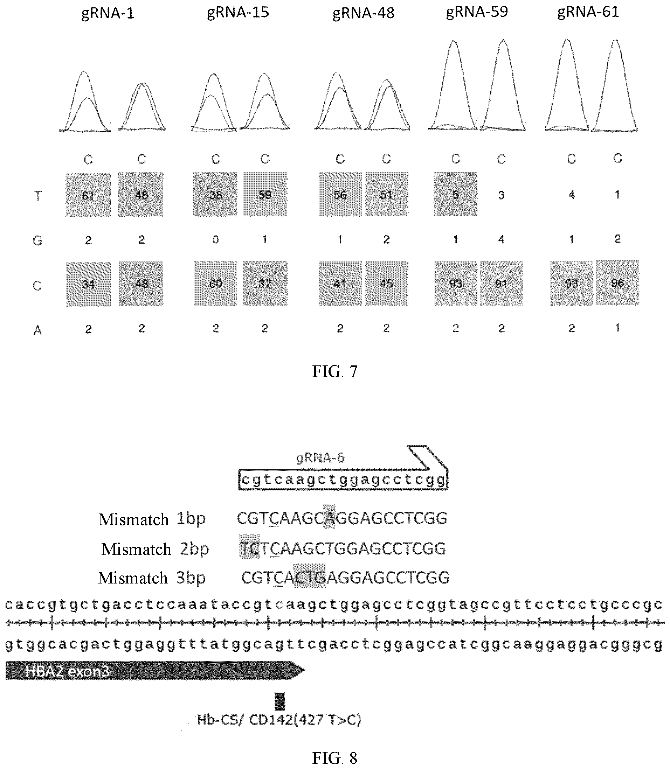

The sequencing results were analyzed for gene editing efficiency using the EditR 10.10 website, as shown in the table below and FIG. 7. FIG. 7 shows a graph of sequencing and efficiency analysis of base editing results at the Hb-CS site, in which the numbers in the boxed areas represent the efficiency (%) of on-target editing.

**Table 8. Repair of Hb-CS mutation by YE1 single base editor**

| Experimental group | Proportion of Hb-CS mutations repaired | |
|---|---|---|
| gRNA-1 | 61% | 48% |
| gRNA-15 | 38% | 59% |
| gRNA-48 | 56% | 51% |
| gRNA-59 | 5% | 3% |
| gRNA-61 | 4% | 1% |

The above experimental results show that the YE1-CBE single base editor mediated by gRNA-1, gRNA-15, and gRNA-48 can repair the Hb-CS mutation of the HBA2 gene, with the repair efficiency significantly superior to that of gRNA-59 and gRNA-61. Additionally, experiments have confirmed that their off-target editing efficiency is much lower than the repair efficiency of the Hb-CS mutation.

Although gRNA-2 and gRNA-5 guide the YE1-CBE single base editor to repair the Hb-CS mutation less efficiently compared to the SpRY single base editor, they can significantly reduce the frequency of off-target editing, lowering the potential risk of off-target editing with the advantage of high safety.

Although the single base editing efficiency of gRNA-15 is lower than that of gRNA-48, the off-target editing efficiency of gRNA-48 is significantly higher than that of gRNA-15 and gRNA-1.

### Example 8

Experiment on repairing Hb-CS mutation using single base editors mediated by gRNAs of different lengths but with the same PAM site.

### 1. Method

In this example, referring to the method of plasmid electroporation in Example 3, plasmids with a mismatch of 1, 2, and 3 nucleotides with gRNA-6 (corresponding to gRNA-12, gRNA-13, and gRNA-14) were respectively mixed with YE1-BE4max-NG (Addgene Plasmid #138159) and electroporated into K562 cell lines. After the incubation of transfected cells for 72 hours, the host cells co-transfected with the single base editor plasmid and gRNA plasmid were recovered. The genes were extracted, and the target fragment was amplified by PCR. The PCR product was subjected to Sanger sequencing.

### 2. Results

The sequencing results were analyzed for gene editing efficiency using the EditR 10.10 website, as shown in the table below and FIG. 8. FIG. 8 shows a schematic diagram of the gRNA guide sequence mismatch, in which the shaded bases represent the mismatched bases and the underlined bases represent the Hb-CS mutation site.

**Table 9. gRNAs with a mismatch of 1 bp to 3 bp all have base repair efficiency**

| gRNA | Number of mismatched bases | Mismatch position (distance from PAM position) | Repair efficiency |
|---|---|---|---|
| gRNA-12 | 1 | 11 | 45% |
| gRNA-13 | 2 | 18, 19 | 42% |
| gRNA-14 | 3 | 12, 13, 14 | 30% |

The above experimental results show that gRNA-6 with a mismatch of 1 to 3 nucleotides can repair the Hb-CS mutation of the HBA2 gene. It can be seen that gRNAs with a mismatch of 1 to 3 nucleotides can still guide the single base editor to repair the Hb-CS mutation of the HBA2 gene.

### Example 9

Experiment on repairing Hb-CS mutation in patient-derived hematopoietic stem/progenitor cells.

### 1. Ex vivo repair experiment

### 2.1 Method

CD34-positive cells (mPBSCs) containing the Hb-CS mutation of the HBA2 gene were obtained and thawed, followed by incubation for two days in StemSpan serum-free expansion medium (StemCell Technologies, StemSpan ^{™} SFEM #09600) enriched with human cytokines (SCF, TPO, and Flt3L, each at 100 ng/mL).

The CBE single base editor and gRNA-1 for repairing the Hb-CS mutation were delivered to mPBSCs. After electroporation, the cells were incubated for one day in SFEM enriched with human cytokines (SCF, TPO, and Flt3L, each at 100 ng/mL), followed by induction of differentiation of mPBSCs into red blood cells in three stages. On the 4th day after induced differentiation, 2 × 10⁵ cells were removed and genomic DNA was extracted. The Hb-CS mutation site of the HBA2 gene and adjacent fragments were then amplified by PCR and subjected to Sanger sequencing. The sequencing results were analyzed for gene editing efficiency using Synthego software.

On the 18th day after induced differentiation, the relative expression level of the repaired HBA2 mRNA was detected by relative real-time quantitative qPCR, the percentage of blood proteins was analyzed by hemoglobin high-performance liquid chromatography (HPLC), and the percentage of denucleated cells in the red blood cells after repair and induced differentiation and the cell size were analyzed by flow cytometry. The experimental group using red blood cells after induced differentiation of unrepaired patient-derived hematopoietic stem/progenitor cells was used as a negative control.

### 2.2 Results

Compared to the negative control, the red blood cells obtained by induced differentiation of mPBSCs treated with the CBE single base editor and gRNA-1 for repairing the Hb-CS mutation restored the expression and function of HBA hemoglobin.

### 2. In vivo repair

The CBE single base editor and gRNA-1 for repairing the Hb-CS mutation can be loaded into viral vectors, or encapsulated in lipid nanoparticles, or expressed by engineered cells and secreted by extracellular vesicles to prepare an intravenous infusion preparation, which was delivered to the bone marrow of the organism via intravenous infusion to repair the Hb-CS mutation in hematopoietic stem cells, thereby restoring the expression and function of HBA hemoglobin.

The technical features of the above examples can be combined arbitrarily, and in order to simplify the description, not all possible combinations of the technical features in the above examples are described. However, as long as there is no contradiction in the combinations of these technical features, they should be considered to be within the scope of the present specification.

The above examples only give expression to several embodiments of the present disclosure, and the descriptions thereof are relatively specific and detailed, but should not be construed as limiting the scope of the present disclosure. It should be noted that, for those of ordinary skill in the art, several modifications and improvements can further be made without departing from the concept of the present disclosure, which all fall within the scope of protection of the present disclosure. Therefore, the scope of protection of the present disclosure shall be subject to the appended claims.

## Claims

1. A method for repairing an HBA2 gene mutation by single base editing, comprising the following steps: contacting a single base editor and a gRNA with an HBA2 gene sequence to be edited and repaired, deaminating the cytosine base of mutated CD142 codon of HBA2 gene, to convert cytosine to thymine.

2. A use of the method for repairing an HBA2 gene mutation by single base editing according to claim 1 in the preparation of a medicament for the treatment of α-thalassemia.

3. A composition for repairing an HBA2 gene mutation, comprising a single base editor and a gRNA, wherein
the single base editor comprises a target nucleic acid-binding structural unit and a deaminase structural unit, wherein the deaminase structural unit comprises a cytosine deaminase active domain;
the gRNA comprises a guide sequence and a scaffold sequence that binds to the single base editor, wherein the guide sequence targets the mutated cytosine base at CD142 codon of the HBA2 gene.

4. The composition according to claim 3, wherein the target nucleic acid-binding structural unit is selected from: CRISPR/Cas nuclease, ZFN, TALEN, and Argonaute.

5. The composition according to claim 4, wherein the CRISPR/Cas nuclease is at least one selected from: Cas9, CasX, CasY, Cpfl, C2c1, cas12b2, cas12h, cas12i, cas12g, C2c2, C2c3, C2c4, C2c5, C2c6, C2c7, c2c8, c2c9, and a variant thereof.

6. The composition according to claim 5, wherein the CRISPR/Cas nuclease is selected from: a Cas9 nickase variant.

7. The composition according to claim 5, wherein the CRISPR/Cas nuclease is at least one selected from: a SpRY variant, a NG-nCas9 variant, and a NGG-nCas9 variant.

8. The composition according to claim 3, wherein the deaminase structural unit is selected from: APOBEC1 deaminase, APOBEC2 deaminase, APOBEC3A deaminase, APOBEC3B deaminase, APOBEC3C deaminase, APOBEC3D deaminase, APOBEC3F deaminase, APOBEC3G deaminase, APOBEC3H deaminase, APOBEC4 deaminase, activation-induced deaminase, pmCDA1, and a variant or combination thereof.

9. The composition according to claim 8, wherein the single base editor is a fusion protein, and the target nucleic acid-binding structural unit is organically connected to the deaminase structural unit.

10. The composition according to claim 9, wherein the single base editor is at least one selected from: SpRY-CBE, AncBE, and YE1-CBE.

11. The composition according to claim 3, wherein the guide sequence of the gRNA binds to or is reverse complementary to a target region of Chr16: 173548-173648.

12. The composition according to claim 11, wherein the guide sequence of the gRNA binds to or is reverse complementary to a target region of Chr16: 173589-173613, Chr16: 173588-173612, Chr16: 173586-173610, Chr16: 173590-173614, Chr16: 173587-173611, Chr16: 173590-173609, or Chr16: 173596-173615.

13. The composition according to claim 12, wherein the base at the 3' end of the guide sequence of the gRNA binds to the base at the 5' end of the antisense strand of the target region.

14. The composition according to claim 13, wherein the guide sequence of the gRNA is reverse complementary to or contains 1 to 5 mismatches with Chr16: 173594-173613, Chr16: 173599-173613, Chr16: 173598-173613, Chr16: 173597-173613, Chr16: 173596-173613, Chr16: 173595-173613, Chr16: 173593-173613, Chr16: 173592-173613, Chr16: 173591-173613, Chr16: 173590-173613, Chr16: 173589-173613, Chr16: 173593-173612, Chr16: 173598-173612, Chr16: 173597-173612, Chr16: 173596-173612, Chr16: 173595-173612, Chr16: 173594-173612, Chr16: 173592-173612, Chr16: 173591-173612, Chr16: 173590-173612, Chr16: 173589-173612, Chr16: 173588-173612, Chr16: 173591-173610, Chr16: 173596-173610, Chr16: 173595-173610, Chr16: 173594-173610, Chr16: 173593-173610, Chr16: 173592-173610, Chr16: 173590-173610, Chr16: 173589-173610, Chr16: 173588-173610, Chr16: 173587-173610, Chr16: 173586-173610, Chr16: 173595-173614, Chr16: 173600-173614, Chr16: 173599-173614, Chr16: 173598-173614, Chr16: 173597-173614, Chr16: 173596-173614, Chr16: 173594-173614, Chr16: 173593-173614, Chr16: 173592-173614, Chr16: 173591-173614, Chr16: 173590-173614, Chr16: 173592-173611, Chr16: 173596-173611, Chr16: 173596-173611, Chr16: 173595-173611, Chr16: 173594-173611, Chr16: 173593-173611, Chr16: 173591-173611, Chr16: 173590-173611, Chr16: 173589-173611, Chr16: 173588-173611, Chr16: 173587-173611, Chr16: 173590-173609, Chr16: 173594-173609, Chr16: 173596-173615, or Chr16: 173600-173615.

15. The composition according to claim 13, wherein the guide sequence of the gRNA is reverse complementary to or contains 1 to 5 mismatches with Chr16: 173594-173613, Chr16: 173598-173613, Chr16: 173597-173613, Chr16: 173596-173613, Chr16: 173595-173613, Chr16: 173593-173613, Chr16: 173592-173613, Chr16: 173591-173613, Chr16: 173590-173613, Chr16: 173589-173613, Chr16: 173593-173612, Chr16: 173597-173612, Chr16: 173596-173612, Chr16: 173595-173612, Chr16: 173594-173612, Chr16: 173592-173612, Chr16: 173591-173612, Chr16: 173590-173612, Chr16: 173589-173612, Chr16: 173588-173612, Chr16: 173591-173610, Chr16: 173595-173610, Chr16: 173594-173610, Chr16: 173593-173610, Chr16: 173592-173610, Chr16: 173590-173610, Chr16: 173589-173610, Chr16: 173588-173610, Chr16: 173587-173610, Chr16: 173586-173610, Chr16: 173595-173614, Chr16: 173599-173614, Chr16: 173598-173614, Chr16: 173597-173614, Chr16: 173596-173614, Chr16: 173594-173614, Chr16: 173593-173614, Chr16: 173592-173614, Chr16: 173591-173614, Chr16: 173590-173614, Chr16: 173592-173611, Chr16: 173596-173611, Chr16: 173595-173611, Chr16: 173594-173611, Chr16: 173593-173611, Chr16: 173591-173611, Chr16: 173590-173611, Chr16: 173589-173611, Chr16: 173588-173611, or Chr16: 173587-173611.

16. The composition according to claim 13, wherein the guide sequence of the gRNA is reverse complementary to or contains 1 to 5 mismatches with Chr16: 173594-173613, Chr16: 173598-173613, Chr16: 173597-173613, Chr16: 173596-173613, Chr16: 173595-173613, Chr16: 173593-173613, Chr16: 173592-173613, Chr16: 173591-173613, Chr16: 173590-173613, or Chr16: 173589-173613;
or
the guide sequence of the gRNA is reverse complementary to or contains 1 to 5 mismatches with Chr16: 173593-173612, Chr16: 173597-173612, Chr16: 173596-173612, Chr16: 173595-173612, Chr16: 173594-173612, Chr16: 173592-173612, Chr16: 173591-173612, Chr16: 173590-173612, Chr16: 173589-173612, or Chr16: 173588-173612;
or
the guide sequence of the gRNA is reverse complementary to or contains 1 to 5 mismatches with Chr16: 173591-173610, Chr16: 173595-173610, Chr16: 173594-173610, Chr16: 173593-173610, Chr16: 173592-173610, Chr16: 173590-173610, Chr16: 173589-173610, Chr16: 173588-173610, Chr16: 173587-173610, or Chr16: 173586-173610;
or
the guide sequence of the gRNA is reverse complementary to or contains 1 to 5 mismatches with Chr16: 173595-173614, Chr16: 173599-173614, Chr16: 173598-173614, Chr16: 173597-173614, Chr16: 173596-173614, Chr16: 173594-173614, Chr16: 173593-173614, Chr16: 173592-173614, Chr16: 173591-173614, or Chr16: 173590-173614;
or
the guide sequence of the gRNA is reverse complementary to or contains 1 to 5 mismatches with Chr16: 173592-173611, Chr16: 173596-173611, Chr16: 173595-173611, Chr16: 173594-173611, Chr16: 173593-173611, Chr16: 173591-173611, Chr16: 173590-173611, Chr16: 173589-173611, Chr16: 173588-173611, or Chr16: 173587-173611.

17. The composition according to claim 3, wherein the 3' end of the guide sequence of the gRNA is at a distance of C12 to C18 from the codon cytosine at CD142 of the HBA2 gene sequence.

18. The composition according to claim 17, wherein the 3' end of the guide sequence of the gRNA is at a distance of C13 to C18 from the codon cytosine at CD142 of the HBA2 gene sequence.

19. The composition according to claim 18, wherein the 3' end of the guide sequence of the gRNA is at a distance of C13 to C17 from the codon cytosine at CD142 of the HBA2 gene sequence.

20. The composition according to claim 3, wherein the length of the guide sequence of the gRNA is 16 bp or more.

21. The composition according to claim 20, wherein the length of the guide sequence of the gRNA is 16 to 25 bp.

22. The composition according to claim 21, wherein the length of the guide sequence of the gRNA is 17 to 25 bp.

23. The composition according to claim 3, wherein the target DNA nucleotide sequence of the guide sequence of the gRNA is selected from a basic sequence shown in SEQ ID NO: 1 to SEQ ID NO: 62 and a mismatched sequence comprising 1 to 5 base mismatches with the basic sequence.

24. The composition according to claim 23, wherein the basic sequence is selected from a sequence shown in SEQ ID NO: 1 to SEQ ID NO: 58.

25. The composition according to claim 3, wherein the single base editor is SpRY-CBE, and the basic sequence is selected from a sequence shown in SEQ ID NO: 1, SEQ ID NO: 3 to SEQ ID NO: 15, SEQ ID NO: 17 to SEQ ID NO: 26, SEQ ID NO: 28 to SEQ ID NO: 37, SEQ ID NO: 39 to SEQ ID NO: 48, and SEQ ID NO: 50 to SEQ ID NO: 58.

26. The composition according to claim 3, wherein the single base editor is AncBE, and the basic sequence is selected from a sequence shown in SEQ ID NO: 1 to SEQ ID NO: 14 and SEQ ID NO: 26 to SEQ ID NO: 36.

27. The composition according to claim 26, wherein the basic sequence is selected from a sequence shown in SEQ ID NO: 1, SEQ ID NO: 3 to SEQ ID NO: 14, SEQ ID NO: 26, and SEQ ID NO: 28 to SEQ ID NO: 36.

28. The composition according to claim 3, wherein the single base editor is YE1-CBE, and the basic sequence is selected from a sequence shown in SEQ ID NO: 1 to SEQ ID NO: 25 and SEQ ID NO: 48 to SEQ ID NO: 58.

29. The composition according to claim 28, wherein the basic sequence is selected from a sequence shown in SEQ ID NO: 1, SEQ ID NO: 3 to SEQ ID NO: 15, SEQ ID NO: 16 to SEQ ID NO: 25, SEQ ID NO: 48, and SEQ ID NO: 50 to SEQ ID NO: 58.

30. A gRNA for repairing an HBA2 gene mutation by single base editing, comprising the gRNA according to any one of claims 3 to 31.

31. A cell, comprising the composition for repairing an HBA2 gene mutation according to any one of claims 3 to 29.

32. The cell according to claim 31, wherein the cell is a cell line and/or a primary cell.

33. The cell according to claim 32, wherein the cell is a hematopoietic stem/progenitor cell, an induced pluripotent stem cell, or an erythroid progenitor cell.

34. A reagent for repairing an HBA2 gene mutation, comprising:
1) the single base editor protein or protein composition according to any one of claims 3 to 29, or a nucleotide sequence encoding the single base editor; and
2) the gRNA according to any one of claims 3 to 29, or a nucleotide sequence encoding the gRNA, or a nucleotide composition comprising the nucleotide sequence encoding the gRNA.
